(19) Europäisches Patentamt
European Patent Office
Office européen des brevets

(11) EP 4 317 231 A1

(12) EUROPEAN PATENT APPLICATION
published in accordance with Art. 153(4) EPC

(43) Date of publication:
**07.02.2024 Bulletin 2024/06**

(21) Application number: **22775885.1**

(22) Date of filing: **21.01.2022**

(51) International Patent Classification (IPC):
***C08F 290/06*** *(2006.01)* ***C08F 220/18*** *(2006.01)*
***C08F 265/06*** *(2006.01)* ***C08F 2/50*** *(2006.01)*
***A61K 6/887*** *(2020.01)* ***A61C 13/00*** *(2006.01)*
***B33Y 70/00*** *(2020.01)* ***B33Y 80/00*** *(2015.01)*

(52) Cooperative Patent Classification (CPC):
**A61C 13/00; A61K 6/887; B33Y 70/00; B33Y 80/00; C08F 2/50; C08F 220/18; C08F 265/06; C08F 290/06**

(86) International application number:
**PCT/KR2022/001128**

(87) International publication number:
**WO 2022/203181 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.03.2021 KR 20210036736**

(71) Applicant: **Osstemimplant Co., Ltd.**
**Gangseo-gu**
**Seoul 07789 (KR)**

(72) Inventors:
- **CHOI, Giho**
  **Seoul 07801 (KR)**
- **KIM, Kyoung Rok**
  **Seoul 07801 (KR)**
- **YANG, Bo Mi**
  **Seoul 07609 (KR)**

(74) Representative: **Beck & Rössig**
**European Patent Attorneys**
**Denninger Str. 169**
**81925 München (DE)**

# (54) PHOTO-CURING RESIN COMPOSITION, AND MOLDED ARTICLE MANUFACTURED THEREFROM

(57) Disclosed are a photo-curable resin composition, and a molded article manufactured therefrom, the photo-curable resin composition comprising: 60-80 parts by weight of a (meth)acrylated urethane-based polymer; 10-30 parts by weight of a (meth)acrylate-based compound including at least one $C_3$-$C_{20}$ cyclic alkyl group; and 1-3 parts by weight of a photopolymerization initiator.

EP 4 317 231 A1

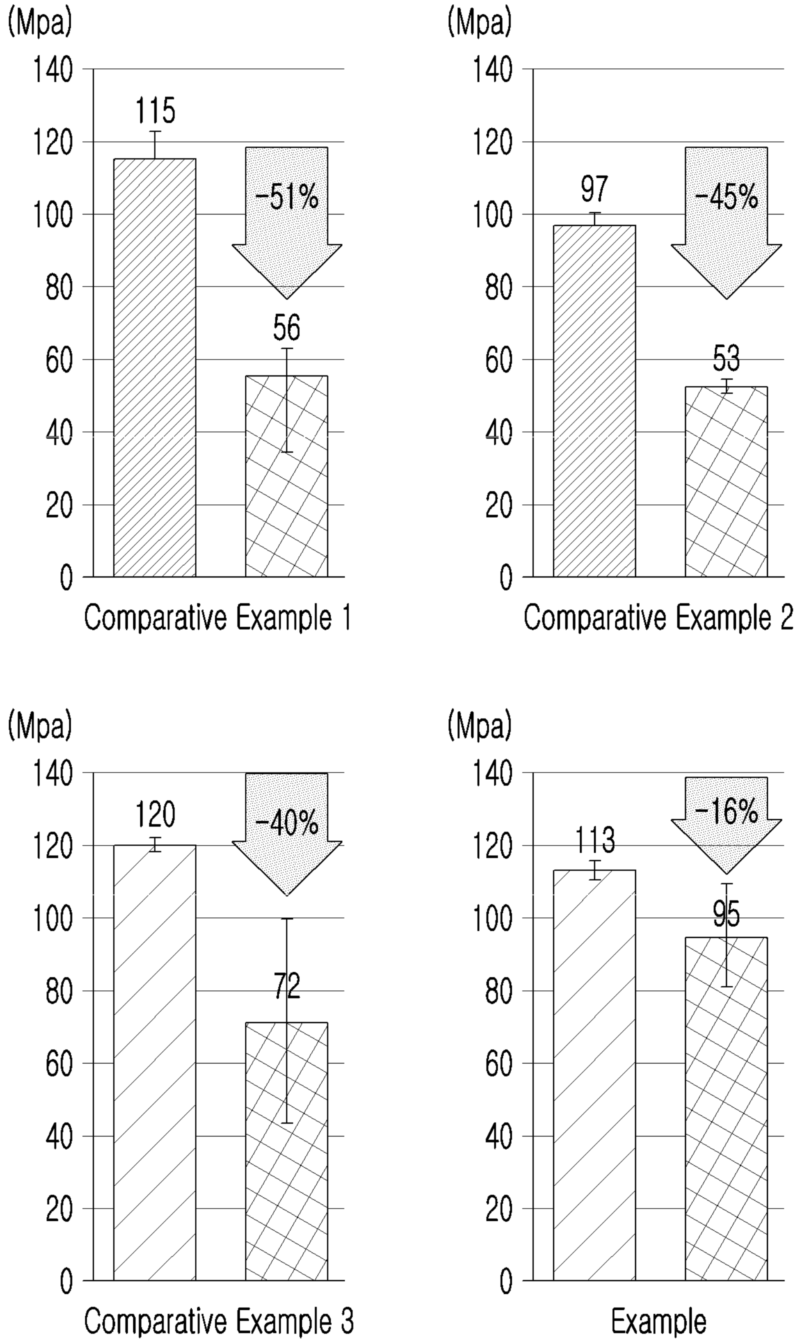
(Mpa)
140
120
100
80
60
40
20
0
115
-51%
56
Comparative Example 1
(Mpa)
140
120
100
80
60
40
20
0
97
-45%
53
Comparative Example 2
(Mpa)
140
120
100
80
60
40
20
0
120
-40%
72
Comparative Example 3
(Mpa)
140
120
100
80
60
40
20
0
113
-16%
95
Example

FIG. 1

## Description

[Technical Field]

The present invention relates to a photo-curable resin composition and a molded article manufactured therefrom.

[Background Art]

3D printers are equipment that manufactures products by processing and laminating materials such as a liquid, powdered resin, metal powder, and solid based on design data. Since 3D printers are capable of easily manufacturing structures with desired shapes, they are used for manufacturing prototypes or structures with a complex shape.

3D printing technology may be divided into a photocuring lamination method, a laser sintering lamination method, a resin extrusion lamination method, an inkjet lamination method, a PolyJet lamination method, and a thin film lamination method according to a material.

The photocuring lamination method is a method of manufacturing a molded article by curing a photo-curable liquid resin with a laser beam or strong ultraviolet rays (UV). As the photocuring lamination method, there are stereolithography apparatus (SLA), digital light processing (DLP), and light induced planar solidification (LIPS).

The laser sintering lamination method is a method of manufacturing a three-dimensional object by sintering powdered materials at high pressure and high temperature using a laser beam. As the laser sintering lamination method, there is selective laser sintering (SLS).

The resin extrusion lamination method is a method of manufacturing a three-dimensional object by extruding wire-shaped materials using an injection head. As the resin extrusion lamination method, there is fused deposition modeling (FDM).

The inkjet lamination method is a method of manufacturing a three-dimensional object by spraying a liquid binder from a printer head nozzle onto materials. As the inkjet lamination method, there is color jetting printing (CJP).

The PolyJet lamination method is a combination of the photocuring method and the inkjet method and is a method of manufacturing a three-dimensional object by spraying materials from a printer head and simultaneously curing the same with ultraviolet rays. As the PolyJet lamination method, there are multi-jet printing (MJP) and PolyJet.

The thin film lamination method is a method of manufacturing a three-dimensional object by cutting thin plate-shaped materials with a precision cutter and then bonding them while heating. As the thin film lamination method, there are laminated object manufacturing (LOM) and paper lamination technology (PLT).

Among the various 3D printing technologies, the photocuring lamination method provides excellent surface roughness characteristics, and thus is suitable for manufacturing dental materials having pores with a complex shape, such as implants. The dental materials need to have stability under moisture conditions due to having intrinsic characteristics such as the use in human teeth and the like. However, a molded article manufactured from a conventional photo-curable resin composition has a problem in which mechanical strength is excessively degraded under moisture. Particularly, since the dental materials such as implants and the like are inevitably exposed to moisture conditions and are subjected to a mechanical external force caused by repetitive mastication, there is a need to develop a technology capable of improving mechanical stability under moisture.

[Disclosure]

[Technical Problem]

The present invention is directed to providing a photo-curable resin composition, in which the degradation of mechanical strength due to moisture is minimized, and a molded article manufactured therefrom.

[Technical Solution]

One aspect of the present invention provides a photo-curable resin composition, which includes: 60 to 80 parts by weight of a (meth)acrylated urethane-based polymer; 10 to 30 parts by weight of a (meth)acrylate-based compound including at least one $C_3$ to $C_{20}$ cyclic alkyl group; and 1 to 3 parts by weight of a photopolymerization initiator.

In an embodiment, the (meth)acrylation may refer to modification of at least one end with one selected from the group consisting of acrylate, methacrylate, methyl acrylate, methyl methacrylate, ethyl acrylate, ethyl methacrylate, propyl acrylate, propyl methacrylate, butyl acrylate, and butyl methacrylate.

In an embodiment, the urethane-based polymer may be urethane dimethacrylate.

In an embodiment, the (meth)acrylate-based compound may be isobornyl acrylate.

In an embodiment, the photopolymerization initiator may be at least one selected from the group consisting of

an acetophenone-based compound, a benzophenone-based compound, a triazine-based compound, a biimidazole-based compound, a thioxanthone-based compound, an oxime ester-based compound, and a phosphine oxide-based compound.

In an embodiment, the photopolymerization initiator may be diphenyl-2,4,6-trimethylbenzoylphosphine oxide or phenylbis(2,4,6-trimethylbenzoyl)-phosphine oxide.

In an embodiment, the photo-curable resin composition may further include 0.1 to 10 parts by weight of a (meth)acrylate-based polymer having an ethylene glycol unit structure.

In an embodiment, the photo-curable resin composition may be a liquid composition used in 3D printing through a digital light processing method, a stereolithography apparatus method, or a light induced planar solidification method.

In an embodiment, the composition may be photo-cured at a wavelength of 360 to 405 nm.

In an embodiment, the composition may have a flexural strength variation under moisture of 30% or less:

[Flexural strength variation under moisture]

$$\text{Flexural strength variation} = (1 - F_2/F_1) \times 100\ (\%)$$

wherein the flexural strength variation under moisture is calculated by the above equation, in which the initial flexural strength after the photo-curable resin composition is photo-cured with a light source having a wavelength of 360 to 405 nm into a form of a disk having a diameter of 10 mm and a thickness of 2 mm is $F_1$, and the flexural strength after the photo-cured composition is immersed in water at 85 °C for 3.3 days is $F_2$.

Another aspect of the present invention provides a molded article manufactured by irradiating the above-described photo-curable resin composition with light.

[Advantageous Effects]

According to an aspect, a photo-curable resin composition, in which the degradation of mechanical strength due to moisture is minimized, and a molded article manufactured therefrom can be provided.

However, it is to be understood that the effects of an aspect of the specification is not limited to the above-described effect but include all effects deducible from the configuration described in the detailed description of the specification or in the claims.

[Description of Drawings]

FIG. 1 shows results of testing mechanical stability under moisture between a molded article manufactured from a photo-curable resin composition according to an example of the specification and a molded article manufactured from a conventional photo-curable resin composition.

[Modes of the Invention]

Hereinafter, an aspect of the specification will be described with reference to the accompanying drawing. However, it should be understood that the descriptions of the specification can be implemented in various other forms, and that it is not intended to limit the present invention to the exemplary embodiments. Also, in the drawing, descriptions of parts unrelated to the detailed description are omitted to clearly describe an aspect of the specification. Throughout the specification, like numbers refer to like elements.

Throughout the specification, the phrase a certain part is "connected" to another part means that the certain part is not only "directly connected" to the other part but also "indirectly connected" to the other part through another member interposed between the two parts. Also, the phrase a certain part "includes" a certain element means that the certain part may further include, instead of excluding, another element unless particularly indicated otherwise.

When a numerical value is presented in the specification, the value has the precision of the significant digit provided in accordance with the standard rules in chemistry for significant digits unless its specific range is stated otherwise. For example, the numerical value 10 includes the range of 5.0 to 14.9 and the numerical value 10.0 includes the range of 9.50 to 10.49.

Hereinafter, an embodiment of the specification will be described in detail with reference to the accompanying drawing.

In the specification, "(meth)acryl-" refers to "methacryl-," "acryl-," or both.

Photo-curable resin composition

A photo-curable resin composition according to an aspect includes: 60 to 80 parts by weight of a (meth)acrylated urethane-based polymer; 10 to 30 parts by weight of a (meth)acrylate-based compound including at least one $C_3$ to $C_{20}$ cyclic alkyl group; and 1 to 3 parts by weight of a photopolymerization initiator.

The photo-curable resin composition can improve a problem in which the mechanical strength of a photo-cured molded article is degraded under moisture.

The (meth)acrylation may refer to modification of at least one end with one selected from the group consisting of acrylate, methacrylate, methyl acrylate, methyl methacrylate, ethyl acrylate, ethyl methacrylate, propyl acrylate, propyl methacrylate, butyl acrylate, and butyl methacrylate. The (meth)acrylated compound may be represented by the following Chemical Formulas.

[Chemical Formulas]

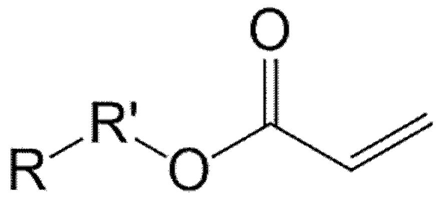

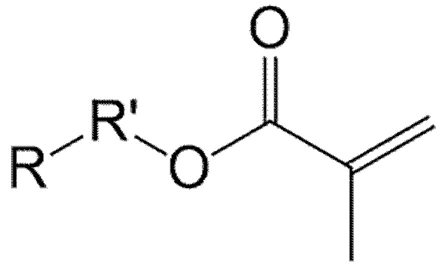

In Chemical Formulas, R is a polymer or compound to be (meth)acrylated, and R' is a single bond, methylene, ethylene, propylene, or butylene. In Chemical Formulas, the left compound refers to an acrylated compound, and the right compound refers to a methacrylated compound.

The content of the urethane-based polymer may be 60 to 80 parts by weight, for example, 60 parts by weight, 62.5 parts by weight, 65 parts by weight, 67.5 parts by weight, 70 parts by weight, 72.5 parts by weight, 75 parts by weight, 77.5 parts by weight, 80 parts by weight, or in a range between two of the above values, and the urethane-based polymer may be urethane dimethacrylate. When the content of the urethane-based polymer is out of the above-described range, the mechanical properties of a molded article may be degraded, or mechanical stability under moisture may be degraded.

The (meth)acrylate-based compound including at least one $C_3$ to $C_{20}$ cyclic alkyl group may be, for example, at least one of cyclopropyl (meth)acrylate, cyclobutyl (meth)acrylate, cyclopentyl (meth)acrylate, cyclohexyl (meth)acrylate, cycloheptyl (meth)acrylate, cyclooctyl (meth)acrylate, cyclononyl (meth)acrylate, cyclodecyl (meth)acrylate, cycloundecyl (meth)acrylate, cyclododecyl (meth)acrylate, cyclotridecyl (meth)acrylate, cyclotetradecyl (meth)acrylate, cyclopentadecyl (meth)acrylate, cyclohexadecyl (meth)acrylate, cycloheptadecyl (meth)acrylate, cyclooctadecyl (meth)acrylate, cyclononadecyl (meth)acrylate, cycloeicosyl (meth)acrylate, and isobornyl (meth)acrylate, but the present invention is not limited thereto. The (meth)acrylate-based compound including at least one cyclic alkyl group can enhance the curing speed and curability of a photo-curable resin composition to improve the mechanical properties of a molded article which is a final product, and can prevent a photopolymerization initiator from being decomposed or eluted upon exposure to moisture. Particularly, strength stability is improved upon curing of the composition, and thus a relative amount of the urethane-based polymer can increase.

The content of the (meth)acrylate-based compound may be 10 to 30 parts by weight, for example, 10 parts by weight, 12.5 parts by weight, 15 parts by weight, 17.5 parts by weight, 20 parts by weight, 22.5 parts by weight, 25 parts by weight, 27.5 parts by weight, 30 parts by weight, or in a range between two of the above values. When the content of the compound satisfies the above-described range, the above-described effects can be realized.

The photopolymerization initiator can initiate polymerization of a (meth)acrylate group in the composition by irradiation with light such as UV rays or the like to form a molded article. The photo-curable resin composition may be cured with a light source having a wavelength of 360 to 405 nm.

The content of the photopolymerization initiator may be 1 to 3 parts by weight, for example, 1 part by weight, 1.25 parts by weight, 1.5 parts by weight, 1.75 parts by weight, 2.0 parts by weight, 2.25 parts by weight, 2.5 parts by weight, 2.75 parts by weight, 3 parts by weight, or in a range between two of the above values. When the content of the photopolymerization initiator satisfies the above-described range, discoloration of a final product can be minimized, and excellent curability can be imparted.

The photopolymerization initiator may be at least one selected from the group consisting of an acetophenone-based compound, a benzophenone-based compound, a triazine-based compound, a biimidazole-based compound, a thioxanthone-based compound, an oxime ester-based compound, and a phosphine oxide-based compound. For example, the photopolymerization initiator may be diphenyl-2,4,6-trimethylbenzoylphosphine oxide or phenylbis(2,4,6-trimethylbenzoyl)-phosphine oxide, but the present invention is not limited thereto.

The photo-curable resin composition may further include a (meth)acrylate-based polymer having an ethylene glycol unit structure. The content of the polymer may be 0.1 to 10 parts by weight, for example, 0.1 parts by weight, 0.5 parts by weight, 1 part by weight, 1.5 parts by weight, 2 parts by weight, 2.5 parts by weight, 3 parts by weight, 3.5 parts by weight, 4 parts by weight, 4.5 parts by weight, 5 parts by weight, 5.5 parts by weight, 6 parts by weight, 6.5 parts by weight, 7 parts by weight, 7.5 parts by weight, 8 parts by weight, 8.5 parts by weight, 9 parts by weight, 9.5 parts by weight, 10 parts by weight, or in a range between two of the above values, but the present invention is not limited thereto. The polymer can prevent an excessive increase in viscosity of the composition due to the addition of high contents of the urethane-based polymer and the (meth)acrylate compound.

The photo-curable resin composition may be a liquid composition used in 3D printing through a digital light processing (DLP) method, a stereolithography apparatus (SLA) method, or a light induced planar solidification (LIPS) method. Therefore, the 3D printing may be performed using a printer using a liquid resin. A photo-curable 3D printer, which is a printer in which a material is cured by irradiating the region to be printed with light, exhibits excellent surface roughness compared to other printing methods and is advantageous for manufacture of a complex structure.

According to the SLA method, a molded article may be manufactured by curing a photo-curable resin composition through projection of UV laser onto a tank containing the photo-curable resin composition and laminating the results. In the SLA 3D printing, the wavelength of a radiated laser may vary depending on the type of composition, and a curing speed, the strength and surface roughness of a cured molded article, and the like may vary.

The DLP method is a mask projection image curing method, and a molded article with a desired shape may be manufactured by curing a photo-curable resin through selective light projection. Unlike a general case in which a product is created as a molding plate moves downward, a product may be created in a downward direction as a molding plate moves upward. In this case, a molded article may be manufactured by projecting the light provided from a beam projector onto a curable resin composition for 3D printing. In other words, a 3D molded product may be manufactured as curing is sequentially performed in units of sliced cross-section layers in the molding plate.

The LIPS method is a method of curing a photo-curable resin through selective projection of a flat plate-shaped light source using an LCD or LED. Unlike the DLP method, the LIPS method can perform uniform photocuring regardless of the area by preventing the bending phenomenon of the wavelength of light according to the curvature of the lens.

The composition may have a flexural strength variation under moisture of 30% or less, for example, 30% or less, 27.5% or less, 25% or less, 22.5% or less, 20% or less, or 17.5% or less:

[Flexural strength variation under moisture]

$$\text{Flexural strength variation} = (1 - F_2/F_1) \times 100\ (\%)$$

wherein the flexural strength variation under moisture is calculated by the above equation, in which the initial flexural strength after the photo-curable resin composition is photo-cured with a light source having a wavelength of 360 to 405 nm into a form of a disk having a diameter of 10 mm and a thickness of 2 mm is $F_1$, and the flexural strength after the photo-cured composition is immersed in water at 85 °C for 3.3 days is $F_2$.

The composition has significantly improved mechanical strength under moisture compared to a conventional photo-curable resin composition, and the flexural strength variation under moisture is merely one physical property criterion expressing these characteristics, and not only is the flexural strength improved.

The photo-curable resin composition may be used in manufacture of various dental materials as it is used in 3D printing. Particularly, according to an embodiment, a photo-curable resin composition satisfying a specific composition may be used to realize excellent strength stability under moisture while satisfying the strength required for dental materials. When the composition of the composition is out of the above-described range, a crosslinked structure may not be sufficiently formed through photocuring alone, and thus desired physical properties may not be satisfied, or stability may be insufficient due to a change in strength under moisture conditions.

Molded article

A molded article according to another aspect is manufactured by irradiating the above-described photo-curable resin composition with light.

The light irradiation may be performed by known SLA, DLP, and LIPS methods.

The molded article may be manufactured by 3D printing through a DLP method, an SLA method, or a LIPS method. Therefore, the molded article may have a smooth surface and a more complex structure compared to a molded article manufactured by a fused deposition modeling in which a filament melts.

In addition, after being cured by light irradiation, the molded article may be post-processed into a shape according to the area of application. For example, the molded article may be a dental material. When a molded article is manufactured by irradiating the photo-curable resin composition with light, the mechanical strength required for a dental material can be sufficiently realized, and stability against moisture can also be improved, and thus the problem of a conventional dental material, in which mechanical strength is excessively reduced under moisture under practical conditions, can be resolved.

Hereinafter, examples of the specification will be described in further detail. However, the following experimental results are obtained from only a few selected examples of the invention, and the scope and contents of the specification should not be interpreted as being reduced or limited by the few selected examples. The effects of each of the various embodiments of the specification, which are not explicitly set forth below, will be described in detail in relevant sections.

Comparative Example 1

31 parts by weight of urethane dimethacrylate, 27 parts by weight of ethoxylated bisphenol A dimethacrylate in which the number of moles of ethylene oxide is 2, 20 parts by weight of polyethylene glycol diacrylate (MW of unit structure: 400), 20 parts by weight of tetrahydrofurfuryl methacrylate, and 1 part by weight of phenylbis(2,4,6-trimethylbenzoyl)-phosphine oxide as a photopolymerization initiator were mixed to prepare a photo-curable composition.

Comparative Example 2

72 parts by weight of urethane dimethacrylate, 20 parts by weight of polyethylene glycol diacrylate (MW of unit structure: 400), 6 parts by weight of isobornyl acrylate, and 1 part by weight of phenylbis(2,4,6-trimethylbenzoyl)-phosphine oxide as a photopolymerization initiator were mixed to prepare a photo-curable composition.

Comparative Example 3

72 parts by weight of urethane dimethacrylate, 6 parts by weight of polyethylene glycol diacrylate (MW of unit structure: 400), 20 parts by weight of tetrahydrofurfuryl methacrylate, and 1 part by weight of phenylbis(2,4,6-trimethylbenzoyl)-phosphine oxide as a photopolymerization initiator were mixed to prepare a photo-curable composition.

Example

72 parts by weight of urethane dimethacrylate, 6 parts by weight of polyethylene glycol diacrylate (MW of unit structure: 400), 20 parts by weight of isobornyl acrylate, and 1 part by weight of phenylbis(2,4,6-trimethylbenzoyl)-phosphine oxide as a photopolymerization initiator were mixed to prepare a photo-curable composition.

Experimental Example

A disk sample having a diameter of 10 mm and a thickness of 2 mm was printed with each composition of the comparative examples and the example using a 3D printer provided with a light source having a wavelength of 360 to 405 nm. The sample was washed with an isopropyl alcohol washing solution and then cured using a post-curing machine for 10 to 30 minutes.

The flexural strength of the sample was measured, and after immersion in water at 85 °C for 3.3 days, flexural strength was measured again. The water immersion condition is an accelerated aging condition that can confirm the same physical properties as those in immersion in water at 37 °C for 3 months.

Flexural strength was measured in accordance with ISO 20795-1, and results are shown in FIG. 1.

Referring to FIG. 1, the example including 60 to 80 parts by weight of the (meth)acrylic urethane copolymer and 10 to 30 parts by weight of the (meth)acrylate-based compound including at least one $C_3$ to $C_{20}$ cyclic alkyl group exhibited excellent strength stability under moisture conditions. However, in the case of Comparative Example 1 including less than 60 wt% of the (meth)acrylic urethane copolymer or Comparative Example 2 including less than 10 parts by weight of the (meth)acrylate-based compound including a cyclic alkyl group, strength stability was not improved.

The foregoing description of the specification is intended for illustration, and it will be understood by those skilled in the art to which the invention pertains that the invention can be easily modified in other specific forms without changing the technical spirit or essential features described in the specification. Therefore, it should be understood that

the examples described above are only exemplary in all aspects and not limiting. For example, each of the constituents described as being one combined entity may be implemented separately, and similarly, constituents described as being separate entities may be implemented in a combined form.

It should be understood that the scope of the specification is defined by the following claims and that all changes or modifications derived from the meaning and scope of the claims and their equivalents are included in the scope of the specification.

## Claims

1. A photo-curable resin composition comprising:

   60 to 80 parts by weight of a (meth)acrylated urethane-based polymer;
   10 to 30 parts by weight of a (meth)acrylate-based compound including at least one $C_3$ to $C_{20}$ cyclic alkyl group; and
   1 to 3 parts by weight of a photopolymerization initiator.

2. The photo-curable resin composition of claim 1, wherein the (meth)acrylation refers to modification of at least one end with one selected from the group consisting of acrylate, methacrylate, methyl acrylate, methyl methacrylate, ethyl acrylate, ethyl methacrylate, propyl acrylate, propyl methacrylate, butyl acrylate, and butyl methacrylate.

3. The photo-curable resin composition of claim 1, wherein the urethane-based polymer is urethane dimethacrylate.

4. The photo-curable resin composition of claim 1, wherein the (meth)acrylate-based compound is isobornyl acrylate.

5. The photo-curable resin composition of claim 1, wherein the photopolymerization initiator is at least one selected from the group consisting of an acetophenone-based compound, a benzophenone-based compound, a triazine-based compound, a biimidazole-based compound, a thioxanthone-based compound, an oxime ester-based compound, and a phosphine oxide-based compound.

6. The photo-curable resin composition of claim 5, wherein the photopolymerization initiator is diphenyl-2,4,6-trimethylbenzoylphosphine oxide or phenylbis(2,4,6-trimethylbenzoyl)-phosphine oxide.

7. The photo-curable resin composition of claim 1, further comprising 0.1 to 10 parts by weight of a (meth)acrylate-based polymer having an ethylene glycol unit structure.

8. The photo-curable resin composition of claim 1, which is a liquid composition used in 3D printing through a digital light processing method, a stereolithography apparatus method, or a light induced planar solidification method.

9. The photo-curable resin composition of claim 1, wherein the composition is photo-cured at a wavelength of 360 to 405 nm.

10. The photo-curable resin composition of claim 1, wherein the composition has a flexural strength variation under moisture of 30% or less:

    [Flexural strength variation under moisture]

    $$\text{Flexural strength variation} = (1 - F_2/F_1) \times 100\ (\%)$$

    wherein the flexural strength variation under moisture is calculated by the above equation, in which the initial flexural strength after the photo-curable resin composition is photo-cured with a light source having a wavelength of 360 to 405 nm into a form of a disk having a diameter of 10 mm and a thickness of 2 mm is $F_1$, and the flexural strength after the photo-cured composition is immersed in water at 85 °C for 3.3 days is $F_2$.

11. A molded article manufactured by irradiating the photo-curable resin composition according to any one of claim 1 to claim 10 with light.

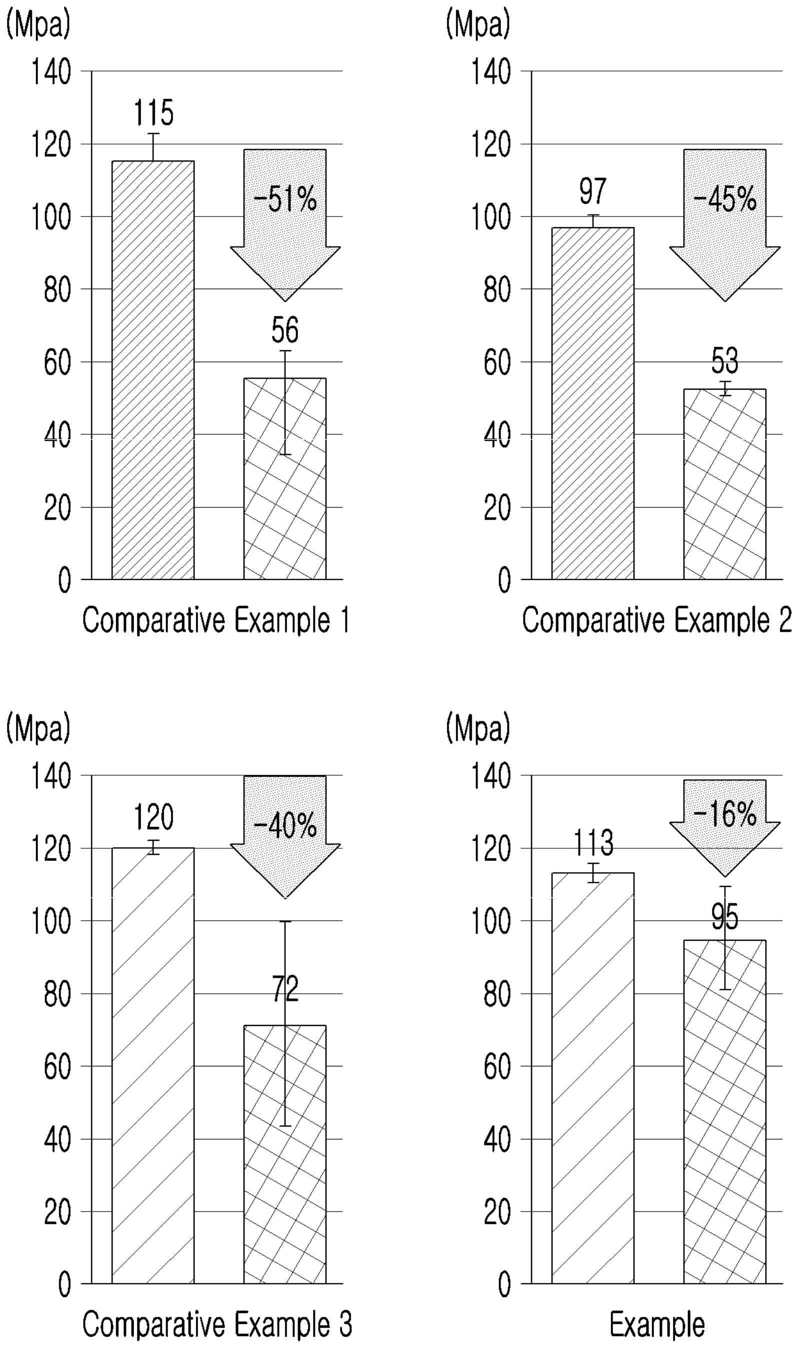
(Mpa)
140
120
100
80
60
40
20
0
115
-51%
56
Comparative Example 1
(Mpa)
140
120
100
80
60
40
20
0
97
-45%
53
Comparative Example 2
(Mpa)
140
120
100
80
60
40
20
0
120
-40%
72
Comparative Example 3
(Mpa)
140
120
100
80
60
40
20
0
113
-16%
95
Example

**FIG. 1**

## INTERNATIONAL SEARCH REPORT

International application No.
**PCT/KR2022/001128**

**A. CLASSIFICATION OF SUBJECT MATTER**

**C08F 290/06**(2006.01)i; **C08F 220/18**(2006.01)i; **C08F 265/06**(2006.01)i; **C08F 2/50**(2006.01)i; **A61K 6/887**(2020.01)i; **A61C 13/00**(2006.01)i; **B33Y 70/00**(2015.01)i; **B33Y 80/00**(2015.01)i

According to International Patent Classification (IPC) or to both national classification and IPC

**B. FIELDS SEARCHED**

Minimum documentation searched (classification system followed by classification symbols)

C08F 290/06(2006.01); A61C 1/08(2006.01); A61C 13/00(2006.01); A61C 7/08(2006.01); A61C 7/12(2006.01); A61K 6/083(2006.01); A61K 6/09(2006.01); B33Y 70/00(2015.01); G03F 7/00(2006.01); G03F 7/027(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 광경화성(photocurable), 3D 프린팅(3D printing), (메트)아크릴화된 우레탄계 중합체((meth)acrylated urethane-based polymer), 사이클릭 알킬기(cyclic alkyl group), 광개시제(photoinitiator), 수분안정성(moisture stability), 굴곡강도(flexural strength)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2020-0056452 A (BASF SE) 22 May 2020 (2020-05-22)<br>See claims 1-40; paragraphs [0003]-[0183]; and table 1. | 1-2,4-6,8-11<br>3,7 |
| Y | KR 10-2016-0087151 A (ELECTRONICS AND TELECOMMUNICATIONS RESEARCH INSTITUTE et al.) 21 July 2016 (2016-07-21)<br>See claim 1; paragraphs [0010]-[0061]; and table 1. | 3,7 |
| A | WO 2020-003169 A1 (3M INNOVATIVE PROPERTIES COMPANY) 02 January 2020 (2020-01-02)<br>See entire document. | 1-11 |
| A | WO 2020-081891 A1 (THE REGENTS OF THE UNIVERSITY OF CALIFORNIA) 23 April 2020 (2020-04-23)<br>See entire document. | 1-11 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 June 2022** | **27 June 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208**<br>Facsimile No. **+82-42-481-8578** | Telephone No. |

Form PCT/ISA/210 (second sheet) (July 2019)

**INTERNATIONAL SEARCH REPORT**

International application No.
**PCT/KR2022/001128**

**C. DOCUMENTS CONSIDERED TO BE RELEVANT**

| Category* | Citation of document, with indication, where appropriate, of the relevant passages | Relevant to claim No. |
|---|---|---|
| A | WO 2020-243429 A1 (ROGERS CORPORATION) 03 December 2020 (2020-12-03)<br>See entire document. | 1-11 |

Form PCT/ISA/210 (second sheet) (July 2019)

**INTERNATIONAL SEARCH REPORT**
**Information on patent family members**

International application No.
**PCT/KR2022/001128**

| Patent document cited in search report | Publication date (day/month/year) | Patent family member(s) | Publication date (day/month/year) |
|---|---|---|---|
| KR 10-2020-0056452 A | 22 May 2020 | CN 111448071 A | 24 July 2020 |
| | | EP 3691901 A1 | 12 August 2020 |
| | | JP 2020-536142 A | 10 December 2020 |
| | | US 2020-0247932 A1 | 06 August 2020 |
| | | WO 2019-070587 A1 | 11 April 2019 |
| KR 10-2016-0087151 A | 21 July 2016 | None | |
| WO 2020-003169 A1 | 02 January 2020 | CN 112367959 A | 12 February 2021 |
| | | EP 3813763 A1 | 05 May 2021 |
| | | JP 2021-529858 A | 04 November 2021 |
| | | KR 10-2020-0130464 A | 18 November 2020 |
| | | KR 10-2302706 B1 | 15 September 2021 |
| | | US 2021-0238328 A1 | 05 August 2021 |
| WO 2020-081891 A1 | 23 April 2020 | CN 113260332 A | 13 August 2021 |
| | | EP 3866726 A1 | 25 August 2021 |
| | | JP 2022-505404 A | 14 January 2022 |
| | | KR 10-2021-0092211 A | 23 July 2021 |
| | | US 2021-0238335 A1 | 05 August 2021 |
| WO 2020-243429 A1 | 03 December 2020 | CN 113906066 A | 07 January 2022 |
| | | EP 3942366 A1 | 26 January 2022 |
| | | KR 10-2022-0016812 A | 10 February 2022 |
| | | US 2020-0377628 A1 | 03 December 2020 |

Form PCT/ISA/210 (patent family annex) (July 2019)